# FASCICULE DE BREVET EUROPEEN

(11) **EP 1 597 197 B1**
(45) Date de publication et mention de la délivrance du brevet: **19.09.2018**
(21) Numéro de dépôt: 04709643.3
(22) Date de dépôt: 10.02.2004
(51) Int. Cl.: C07C 29/74, C07C 31/08, C07C 67/56, B01J 20/18, B01D 15/00

(54) **PROCÉDÉ DE SÉCHAGE D'ESTERS OU D'ALCOOLS AU MOYEN D'ADSORBANTS AGGLOMÉRÉS**
VERFAHREN ZUM TROCKNEN VON ESTERN ODER ALKOHOLEN UNTER VERWENDUNG VON AGGLOMERIERTEN ADSORPTIONSMITTELN
PROCESS FOR DRYING ESTERS OR ALCOHOLS USING AGGLOMERATED ADSORBENTS

(30) Priorité: 11.02.2003 FR 0301596
(43) Date de publication de la demande: 23.11.2005
(73) Titulaire: ARKEMA FRANCE, 92700 Colombes (FR)
(72) Inventeur: PLEE, Dominique, F-64140 Lons (FR)
(74) Mandataire: Schaefer, Anne-Sophie
(86) Numéro de dépôt international: PCT/FR2004/000298
(87) Numéro de publication internationale: WO 2004/071945

(56) Documents cités:
- EP-B1- 1 467 811
- WO-A-00/34217
- WO-A-96/03199
- US-A- 3 634 331
- US-A- 4 726 818
- US-A- 5 008 227
- US-A- 5 045 295
- PATENT ABSTRACTS OF JAPAN vol. 018, no. 134 (C-1176), 4 mars 1994 (1994-03-04) & JP 05 317700 A (TOSOH CORP), 3 décembre 1993 (1993-12-03)
- PATENT ABSTRACTS OF JAPAN vol. 0182, no. 76 (C-1204), 26 mai 1994 (1994-05-26) & JP 06 048728 A (TOSOH CORP), 22 février 1994 (1994-02-22)
- PATENT ABSTRACTS OF JAPAN vol. 004, no. 144 (C-027), 11 octobre 1980 (1980-10-11) & JP 55 092120 A (SHINTOUHOKU KAGAKU KOGYO KK), 12 juillet 1980 (1980-07-12)

## Description

La présente invention concerne un procédé de déshydratation de composés organiques, particulièrement des alcools ou des esters, au moyen d'adsorbants agglomérés à base de tamis moléculaire d'ouverture de pores égale à environ 3 Å.

### Art antérieur

De nombreuses molécules organiques sont produites dans un milieu contenant de l'eau ou synthétisées par hydrolyse. Par exemple, les alcools produits par hydratation d'une oléfine, que ce soit l'éthanol à partir d'éthylène, ou bien d'isopropanol à partir de propylène, contiennent, de l'eau une fois la réaction terminée.

Les alcools obtenus par fermentation de matières agricoles telles que la betterave, le maïs ou la canne à sucre présentent également une importante teneur en eau .

Les esters synthétisés à partir d'alcool et d'acide carboxylique peuvent également être contaminés par des traces d'eau une fois leur synthèse terminée.

Or, de nombreuses utilisations des composés organiques nécessitent qu'ils soient exempts ou au pire ne contiennent que d'infimes traces d'eau, telles que des applications dans le domaine pharmaceutique ; il est nécessaire de trouver des méthodes efficaces pour éliminer le maximum d'eau.

Il existe des méthodes de déshydratation par passage du composé organique à déshydrater au travers d'un lit d'adsorbant (dessicant).

Pour le séchage de molécules organiques selon ces méthodes, Il est connu d'utiliser comme adsorbants des zéolites dont l'ouverture de pores est de l'ordre de 3 Å où l'eau peut théoriquement pénétrer à la différence des molécules organiques de taille supérieure. Parmi les zéolites dont l'ouverture des pores est de l'ordre de 3 Å, on peut par exemple citer les zéolites de type A dont 28 à 60 % (rapportés en équivalents) des sites cationiques échangeables sont occupés par des ions potassium, le reste des sites étant essentiellement occupé par Na⁺, appelées aussi zéolites 3A.

Les zéolites se présentant sous forme de cristaux de très petite taille, typiquement inférieure à 10 µm, leur utilisation nécessite le plus souvent une mise en sous forme d'objets, tels que billes, filés ou extrudés, de granulométrie plus importante, typiquement comprise entre 0,5 mm et 5 mm et ce, afin notamment d'éviter les pertes de charge dues lors des manipulations de ces tamis, notamment au cours des opérations de chargement et de déchargement des colonnes d'adsorption. La mise en forme de ces objets, appelés dans tout ce qui suit agglomérés, est généralement effectuée au moyen de liants, notamment argileux parmi lesquels on peut citer la sépiolite, l'attapulgite, la montmorillonite ou les argiles de la famille des kaolins.

FR 2.495.007 ou GB 2.088.739 décrivent un procédé de déshydratation de solvants organiques par passage lent (vitesse superficielle du solvant dans la colonne inférieure à 15 cm/min) au travers d'une colonne contenant un tamis moléculaire déshydraté à base de zéolite 3 A où le rapport entre la longueur de la colonne et la zone de transfert de masse est supérieur ou égal à 4.

US 4.407.662 décrit un procédé d'adsorption de l'eau de type VPSA (mis pour Vacuum Swing Adsorption ce que l'on traduit en français par adsorption modulée sous vide) comprenant une étape d'adsorption en phase gaz en sortie de distillation sur une colonne de tamis moléculaire 3 A, suivie d'une étape de régénération à pression inférieure à la pression d'adsorption avec purge d'une partie de l'éthanol anhydre . L'intérêt de ce procédé d'adsorption est qu'il permet d'effectuer des cycles beaucoup plus rapides puisque l'ensemble du procédé est normalement isotherme, exception faite des chaleurs d'adsorption et désorption qui génèrent une variation de température que la demanderesse évalue à moins de 14°C mais la capacité d'adsorption utilisée est très inférieure a la capacité à saturation.

Un des inconvénients constatés lors de l'utilisation de tamis moléculaires 3 A commerciaux classiques agglomérés pour la déshydratation de composés organiques, que ce soit en phase gaz ou en phase liquide, concerne la formation de molécules indésirables catalysées par le tamis moléculaire. Dans le cas de la déshydratation de l'éthanol, on observe la formation d'acétaldéhyde, d'éthylène, de diéthyléther voire même de molécules plus complexes comme le paraldéhyde formé par condensation cyclique d'acétaldéhyde, les acétals et hémiacétals, formés par réaction de l'éthanol sur l'acétaldéhyde.

Ces réactions parasites dépendent également de la température et donc de la pression d'adsorption qui, dans les procédé PSA ou VPSA, est imposée (il faut éviter la condensation liquide).

Outre la quantité d'eau qui doit être très faible, certaines applications des composés organiques, notamment dans le domaine pharmaceutique, exigent des composés organiques de très haute pureté où l'ensemble des espèces indésirables organiques ne dépasse pas 10 ppm.

WO 00/34217 décrit un procédé de séchage de liquides organiques où l'on traite ce liquide à déshydrater au moyen d'un tamis moléculaire 3 A ayant préalablement subi un traitement destiné à réduire sa concentration en sites acides à moins de 18 mmoles/g, mesurée par TPD (mis pour Temperature Programmed Desorption ou en français, Désorption en Température Programmée) de NH₃. Le prétraitement consiste à la mise en contact du tamis moléculaire avec une solution de sel de métal alcalin, de préférence de nitrate de potassium, suivie de plusieurs lavages. Grâce à ce prétraitement effectué sur deux tamis 3 A commerciaux classiques agglomérés, WO 00/34217 montre que l'on parvient à diminuer d'une part la formation de propylène lors du séchage de l'isopropanol et d'autre part la formation de diéthyléther lors du séchage de l'éthanol. Outre le fait que ce procédé fait appel à plusieurs étapes de contact entre un solide et un liquide, ce qui le complique et augmente son coût, il est limité dans sa capacité à diminuer fortement la teneur en sites acides du tamis moléculaire, responsables des réactions acides, telles que la déshydratation intramoléculaire ou intermoléculaire ou encore la formation d'acétal. A l'appui de cette remarque on se reportera plus particulièrement aux exemples figurant dans WO 00/34127 où l'abaissement de la teneur en sites acides de deux zéolites commerciales est inférieur à 50%.

### Description de l'invention

La présente invention concerne un procédé de séchage d'esters ou d'alcools, en phase gaz ou en phase liquide, par passage au travers d'un lit d'adsorbant à base de tamis moléculaires agglomérés à base de zéolite 3 A et d'un ou plusieurs liants d'agglomération, lesdits tamis moléculaires agglomérés étant caractérisés en ce que leur teneur en fer, exprimée en Fe2O3 par rapport à la masse anhydre de tamis, est inférieure ou égale à 0,5 %, et de préférence inférieure ou égale à 0,3 %, en poids et leur teneur en titane, exprimée en TiO2 par rapport à la masse anhydre totale de tamis est inférieure ou égale à 0,2%, de préférence inférieure ou égale à 0,1%. L'invention concerne notamment un procédé de séchage d'éthanol. Les tamis moléculaires à base de zéolite 3 A agglomérés, lorsqu'ils sont utilisés dans un procédé de séchage de composés organiques liquides ou gazeux par passage du ou des composés à déshydrater sur un lit à base desdits tamis agglomérés présentent l'avantage de limiter la formation d'espèces indésirables obtenues par conversion partielle du ou des composés organiques à sécher.

Les tamis agglomérés utilisés dans le procédé de l'invention sont de granulométrie moyenne comprise en général entre 1,6 mm et 5 mm.

Ils peuvent être préparés par agglomération, selon les techniques connues, de poudre de zéolite, obtenue par exemple par synthèse hydrothermale, avec un liant d'agglomération choisi parmi les argiles telles que les kaolins, la silice et/ou l'alumine. En général, les tamis agglomérés utilisés dans le procédé de l'invention contiennent moins de 25 % de liant inerte (au sens de l'adsorption) et de préférence jusqu'à 20 % en poids, avantageusement jusqu'à 10 % en poids et encore plus avantageusement voisin de 5%.

Les liants qui conviennent pour la présente invention seront choisis parmi les liants d'agglomération usuels ; l'homme du métier sélectionnera aisément ceux dont les teneurs en fer et en titane permettront d'obtenir les tamis agglomérés utilisés dans le procédé selon l'invention.

L'agglomération peut, par exemple, être menée par mélange d'une poudre cristalline de zéolite (ici 3 A ou 4A) avec de l'eau, le liant (généralement également sous forme de poudre) et éventuellement des additifs d'aide à l'agglomération puis extrusion ou pressage du mélange ainsi obtenu sous forme de filés ou bien pulvérisation de ce mélange sur des agglomérats de zéolites jouant le rôle de germe d'agglomération. Pendant la pulvérisation, les agglomérats de zéolite sont soumis à une rotation continue sur eux-mêmes. Ceci peut être réalisé en disposant les agglomérats dans un réacteur en rotation sur lui-même autour d'un axe de rotation, ledit axe de rotation étant préférablement incliné par rapport à la direction verticale. Par ce procédé, couramment désigné dans la technique par procédé « boule de neige » on obtient des agglomérats sous forme de billes.

Les tamis agglomérés ainsi mis en forme sont ensuite soumis à une cuisson à une température comprise entre environ 400 et 700 °C.

Une variante destinée à l'obtention de tamis agglomérés à faible taux de liant inerte consiste à agglomérer la poudre de zéolite avec un liant zéolitisable comme indiqué ci-dessus, puis à zéolitiser le liant puis à laver et sécher le produit obtenu et l'activer à une température comprise entre 400 et 700 °C.

Le liant zéolitisable peut être choisi parmi les argiles zéolitisables telles que le kaolin, le métakaolin, l'halloysite, seules ou en mélange.

L'étape de zéolitisation consiste à convertir tout ou partie du liant zéolitisable avec lequel on a préalablement aggloméré la poudre zéolite par macération alcaline.

Les tamis sont de manière préférentielle soumis à un traitement qui consiste à introduire une espèce basique :
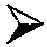 soit par imprégnation en phase aqueuse des tamis 3 A agglomérés et cuits au moyen d'hydroxyde(s) de métal(aux) alcalin(s) à température ambiante (15-30 °C) ou de tamis 4 A, agglomérés et cuits, puis traités en phase aqueuse au moyen d'hydroxyde(s) de métal(aux) alcalin(s) à une température comprise entre 70 °C et l'ébullition, suivie de lavages pour éliminer les espèces ioniques en excès puis d'un échange au potassium et d'un séchage,
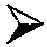 soit par incorporation de ce(s) d'hydroxyde(s) et/ou carbonate(s) et/ou de sel(s) de métal(aux) alcalin(s) et d'acide(s) organique(s) comme acétate, lactate, oxalate, citrate,..., lors de l'étape d'agglomération et de mise en forme. Cette seconde variante, particulièrement préférée par la demanderesse, ne nécessite pas d'opérations de remouillage et de lavage du tamis après mise en forme.

Les tamis utilisés dans le procédé selon l'invention qui ont été soumis à ce dernier traitement ont de préférence une teneur en métal alcalin (de préférence sodium et/ou potassium), exprimée en M₂O, en excédent de la capacité d'échange de la zéolite (M= de préférence Na et/ou K) par rapport à la masse anhydre totale de l'aggloméré, supérieure à égale à 0,5 % et inférieure ou égale à 1,4 %, et de préférence supérieure à égale à 0,7 % et inférieure ou égale à 1,1 %.

### Exemples - description du montage expérimental

On teste la capacité à favoriser la formation d'acétaldéhyde lors d'une opération de séchage d'éthanol sur des tamis agglomérés à base de zéolite 3A (zéolite de type A dont 46 % de la CEC (capacité d'échange cationique) est occupée par des ions potassium, le reste étant occupé par des ions sodium et agglomérés avec 20 % en poids (par rapport à la masse totale de l'aggloméré) de différents liants se présentant sous forme d'extrudés de granulométrie moyenne d'environ 1,6 mm à l'aide du montage constitué des éléments suivants :
- une réserve d'éthanol à 96 % en poids (les 4 % restants étant de l'eau) reliée à une pompe péristaltique,
- un réacteur cylindrique vertical (volume = 200 ml) placé dans un four alimenté par la pompe ; ce réacteur est chargé jusqu'au tiers de sa hauteur par des billes de verre inertes vis-à-vis des réactions considérées et contient au dessus le tamis moléculaire à tester, soit une quantité de l'ordre de 70 ml,
- une arrivée d'azote permettant de purger le réacteur de l'air qui y est initialement contenu,
- un condenseur en sortie de réacteur.

Le réacteur est chargé de tamis puis balayé à l'azote pendant 1 heure ; on programme la montée en température du four de façon à atteindre 140 °C. On pompe alors l'éthanol sous forme liquide qui se vaporise dans le réacteur et se recondense en sortie (température = 14 ° C)

On effectue ensuite des analyses par chromatographie en phase gazeuse (CPG) des fractions obtenues pour doser les quantités formées.

### EXEMPLE 1

On teste plusieurs tamis agglomérés pour leur capacité à convertir l'éthanol en acétaldéhyde et l'on reporte au tableau 1 les teneurs pondérales en éléments mineurs de ces tamis ainsi que leur taux de conversion d'éthanol en acétaldéhyde.

Le tamis 1 contient 20 parties en poids de liant qui contient calcium, fer, magnésium et titane dans les proportions suivantes : CaO = 0,09 % ; Fe₂O₃ = 0,77 % ; MgO = 1,15 % ; TiO₂ = 0,1 % ; le tamis 2 contient 20 parties en poids de liant qui contient calcium, fer, magnésium et titane dans les proportions suivantes : CaO = 0,6 % ; Fe₂O₃ = 4,7 % ; MgO = 12, 5 % ; TiO₂ = 0,5 %. et le tamis 3 contient 20 parties en poids de liant qui contient calcium, fer, magnésium et titane dans les proportions suivantes : CaO = 0 % ; Fe₂O₃ = 1,3 % ; MgO = 0,2 % ; TiO₂ = 2 %.

Le tamis 4 est obtenu à partir du tamis 1 en imprégnant 50 g de tamis 1 par 40 ml d'une solution aqueuse contenant 12,64 g de nitrate de fer nonahydrate ; cette quantité correspond à 2,5 g de Fe₂O₃ retenus sur le solide.

**Tableau 1**

| Tamis aggloméré | CaO (%) | MgO (%) | Fe₂O₃ (%) | TiO₂ (%) | Conversion (ppm) |
|---|---|---|---|---|---|
| 1 selon l'invention | 0,018 | 0,23 | 0,15 | 0,018 | 0 |
| 2 comparatif | 0,12 | 2,5 | 0,94 | 0,1 | 50 |
| 3 comparatif | 0 | 0,04 | 0,26 | 0,4 | 7 |
| 4 comparatif | 0,017 | 0,21 | 5 | 0,017 | 50 |

### EXEMPLE 2

On teste plusieurs tamis pour leur capacité à former de l'éthylène et du diéthyléther lors de la déshydratation de l'éthanol et l'on reporte au tableau 2 les teneurs pondérales en éléments mineurs de ces tamis agglomérés ainsi que les résultats des tests catalytiques.

Le tamis 5 contient 20 parties en poids de liant qui contient calcium, fer, magnésium et titane dans les proportions suivantes : CaO = 1 % ; Fe₂O₃ = 0,95 % ; MgO = 5,65 % ; TiO₂ = 0,05 %.

Le tamis 6 est obtenu par imprégnation de 40 g de tamis 5 par 40 ml d'une solution aqueuse de potasse contenant 0,8 g de KOH sans lavage à l'eau. La quantité résiduelle de potassium sur le tamis 6, exprimée en quantité de K₂O, est de 0,8 %.

Le tamis 7 est obtenu par incorporation au moment de l'étape d'agglomération de 0,9 g de K₂CO₃ pour 41 g de poudre de zéolite et 9 g de liant. La quantité résiduelle de potassium sur le tamis 7, exprimée en quantité de K₂O, est de 1,16 %.

On mesure également la quantité d'acétaldéhyde formée sur les tamis 5 à 7 ; pour ces trois échantillons, elle est de l'ordre de 3 ppm.

**Tableau 2**

| Tamis | CaO (%) | MgO (%) | Fe₂O₃ (%) | TiO₂ (%) | K₂O résiduel (%) | Conversion Ether (ppm) | Conversion Ethylène (ppm) |
|---|---|---|---|---|---|---|---|
| 5 selon l'invention | 0,2 | 1,13 | 0,19 | 0,01 | 0 | 15 | 15 |
| 6 selon l'invention | 0,2 | 1,13 | 0,19 | 0,01 | 0,8 | 2 | 2 |
| 7 selon l'invention | 0,2 | 1,13 | 0,19 | 0,01 | 1,13 | 3 | <1 |

### EXEMPLE 3

On met sous forme d'extrudés de 1,6 mm de diamètre un tamis en agglomérant 80% de poudre 4 A avec 20 % de liant zéolitisable contenant du calcium du fer, du magnésium et du titane dans les proportions suivantes : CaO = 0,09 % ; Fe₂O₃ = 0,77 % ; MgO = 1,15 % ; Ti0₂ = 0,1 %

Après calcination à 550 °C pendant 2 h, on immerge la moitié du tamis dans une solution de soude à 100 °C pendant 2 h (L/S = 4 ; NaOH = 80 g/l), puis on lave le tamis à l'eau jusqu'à pH = 11 , et on opère un échange au potassium de façon à obtenir un taux d'échange équivalent à l'exemple 1 (tamis 8).

Une mesure de la capacité d'adsorption de H₂O sur ce tamis sous pression partielle de 0,5 à une température de 25 °C montre une amélioration d'environ 20 % par rapport au tamis n'ayant pas subi le traitement en milieu basique à 100 °C (le liant inerte représente 4 % de la masse totale de l'aggloméré final)

On effectue le test catalytique décrit dans les exemples précédents sur les deux tamis, ayant ou non subi le traitement dans la solution de soude/potasse et on trouve les valeurs indiquées dans le tableau ci-dessous.

**Tableau 3**

| Conversion (ppm) | sans traitement basique tamis 1 | avec traitement basique tamis 8 |
|---|---|---|
| Acétaldéhyde (%) | 0 | 0 |
| Ether (%) | 7 | 3 |
| Ethylène (%) | 20 | 8 |

Une variante de ce procédé consiste à agglomérer 80 % d'une poudre 3A avec 20 % de liant zéolitisable, à cuire, puis à immerger le solide dans une solution de NaOH (80 g/l) + KOH (30 g/l), pendant 2 h à 100 °C , à laver pour débarrasser le solide de ses sels excédentaires et à le sécher à 80 °C. La capacité d'adsorption d'eau, déterminée dans les mêmes conditions que précédemment est augmentée de 13 % par rapport au tamis n'ayant pas subi de traitement en milieu basique à 100 °C (le liant inerte représente 8 % de la masse totale de l'aggloméré final)

## Revendications

1. Procédé de séchage d'esters ou d'alcools, en phase gaz ou en phase liquide, par passage au travers d'un lit d'adsorbant à base de tamis moléculaires agglomérés à base de zéolite 3 A et d'un ou plusieurs liants d'agglomération, lesdits tamis moléculaires agglomérés étant **caractérisés en ce que** leur teneur en fer, exprimée en Fe₂O₃ par rapport à la masse anhydre de tamis, est inférieure ou égale à 0,5 %, et de préférence inférieure ou égale à 0,3 %, en poids et leur teneur en titane, exprimée en Ti0₂ par rapport à la masse anhydre totale de tamis est inférieure ou égale à 0,2%, de préférence inférieure ou égale à 0,1%.

2. Procédé selon la revendication 1, de séchage d'éthanol.

3. Procédé selon la revendication 1 ou la revendication 2, **caractérisé en ce que** la granulométrie moyenne desdits tamis moléculaires agglomérés est comprise entre 1,6 mm et 5 mm.

4. Procédé selon l'une quelconque des revendications précédentes, **caractérisé en ce que** la teneur desdits tamis moléculaires agglomérés en métal(aux) alcalin(s), exprimée en M₂O, en excédent de la capacité d'échange de la zéolite (M étant de préférence le sodium et/ou le potassium) par rapport à la masse anhydre totale de tamis est supérieure ou égale à 0,5% et inférieure ou égale à 1,4%, et de préférence supérieure ou égale à 0,7% et inférieure ou égale à 1,1%.

5. Procédé selon l'une quelconque des revendications précédentes, **caractérisé en ce que** les tamis moléculaires agglomérés contiennent moins de 25 % en poids de liant inerte, de préférence au plus 20%, avantageusement au plus 10 % et encore plus avantageusement environ 5%.

## Patentansprüche

1. Verfahren zum Trocknen von Estern oder Alkoholen in Gasphase oder in Flüssigphase durch Durchströmen eines Adsorptionsmittelbetts, das aus agglomerierten Molekularsieben, die aus Zeolit 3A bestehen, und einem oder mehreren Agglomerationsbindemitteln besteht,
wobei die agglomerierten Molekularsiebe **dadurch gekennzeichnet sind, dass** ihr Eisengehalt, ausgedrückt in Fe₂O₃ bezogen auf die wasserfreie Siebmasse, kleiner oder gleich 0,5 Gew.-% und vorzugsweise kleiner oder gleich 0,3 Gew.-% ist, und ihr Titangehalt, ausgedrückt in TiO₂ bezogen auf die gesamte wasserfreie Siebmasse kleiner oder gleich 0,2 %, vorzugsweise kleiner oder gleich 0,1 % ist.

2. Verfahren nach Anspruch 1 zum Trocknen von Ethanol.

3. Verfahren nach Anspruch 1 oder Anspruch 2, **dadurch gekennzeichnet, dass** die mittlere Teilchengröße der agglomerierten Molekularsiebe im Bereich zwischen 1,6 mm und 5 mm liegt.

4. Verfahren nach einem der vorhergehenden Ansprüche, **dadurch gekennzeichnet, dass** der Gehalt der agglomerierten Molekularsiebe an Alkalimetall(en), ausgedrückt in M₂O, im Überschuss der Austauschkapazität des Zeoliten (wobei M vorzugsweise Natrium und/oder Kalium ist) bezogen auf die gesamte wasserfreie Siebmasse größer oder gleich 0,5 % und kleiner oder gleich 1,4 %, und vorzugsweise größer oder gleich 0,7 % und kleiner oder gleich 1,1 % ist.

5. Verfahren nach einem der vorhergehenden Ansprüche, **dadurch gekennzeichnet, dass** die agglomerierten Molekularsiebe weniger als 25 Gew.-%, vorzugsweise höchsten 20 %, vorzugsweise höchstens 10 % und noch bevorzugter etwa 5 % inertes Bindemittel enthalten.

## Claims

1. Process for drying esters or alcohols, in the gas phase or in the liquid phase, by passing through a bed of adsorbent based on agglomerated molecular sieves based on zeolite 3A and on one or more agglomeration binders, said agglomerated molecular sieves being **characterized in that** their content of iron, expressed as Fe₂O₃, with respect to the anhydrous weight of sieves is less than or equal to 0.5% and preferably less than or equal to 0.3%, by weight, and their content of titanium, expressed as TiO₂, with respect to the total anhydrous weight of sieves is less than or equal to 0.2%, preferably less than or equal to 0.1%.

2. Process according to Claim 1, for drying ethanol.

3. Process according to Claim 1 or Claim 2, **characterized in that** the mean particle size of said agglomerated molecular sieves is between 1.6 mm and 5 mm.

4. Process according to any one of the preceding claims, **characterized in that** the content, in said agglomerated molecular sieves, of alkali metal(s), expressed as M₂O, surplus to the exchange capacity of the zeolite (M preferably being sodium and/or potassium), with respect to the total anhydrous weight of sieves is greater than or equal to 0.5% and less than or equal to 1.4% and preferably greater than or equal to 0.7% and less than or equal to 1.1%.

5. Process according to any one of the preceding claims, **characterized in that** the agglomerated molecular sieves contain less than 25% by weight of inner binder, preferably at most 20%, advantageously at most 10% and more advantageously still approximately 5%.
